# EUROPEAN PATENT SPECIFICATION

(11) **EP 4 076 223 B1**
(45) Date of publication and mention of the grant of the patent: **11.03.2026**
(21) Application number: 20839254.8
(22) Date of filing: 16.12.2020
(51) Int. Cl.: A61B 17/22, A61M 25/00

(54) **ELECTROLYTIC REMODELING OF CHRONIC CLOT**
ELEKTROLYTISCHE UMBAU VON CHRONISCHEN OBSTRUKTIONEN
REMODELAGE ELECTROLYTIQUE D'OBSTRUCTION CHRONIQUE

(30) Priority: 20.12.2019 US 201962951294 P
(43) Date of publication of application: 26.10.2022
(73) Proprietor: Koninklijke Philips N.V., 5656 AG Eindhoven (NL)
(72) Inventor: ROLF, David, 5656 AE Eindhoven (NL); DECKER, Cedar, 5656 AE Eindhoven (NL); TSCHIDA, Adam, 5656 AE Eindhoven (NL)
(74) Representative: Philips Intellectual Property & Standards
(86) International application number: PCT/EP2020/086485
(87) International publication number: WO 2021/122790

(56) References cited:
- CN-A- 108 452 426
- CN-A- 109 223 100
- US-A1- 2015 342 681
- US-A1- 2017 056 035

## Description

### FIELD OF THE DISCLOSURE

The present disclosure relates generally to the use of medical devices for the treatment of chronic venous disease and collagenous scar tissue. In particular, the present disclosure provides systems for remodeling a collagen obstruction using energy and an expandable catheter.

### BACKGROUND

Chronic venous disease is a condition in which clots form and remain in the venous circulatory system, especially in the lower extremities, such as the lower portion of the leg below the knee. In healthy people, young clots in an artery or vein (also known as venous thrombus) are primarily made of fibrin and will generally resolve via the action of natural lytics in the circulatory system and normal physical activity. Barring this resolution, however, the longer a clot persists, the more likely that it may cause harm, especially if it develops into an embolus and travels through the bloodstream or becomes a chronic clot and is wedged like a scar in the vein. When a clot persists for longer than about 30 days, its composition changes from primarily fibrinous (capable of dissolving via natural lytics) to crosslinked collagen. Crosslinked collagen is rubbery, elastic and tough, and a chronic clot can attach strongly to the vein wall via tendrils called synechiae. Once developed, a chronic clot can drastically reduce venous blood flow, causing significant negative symptoms. Conventional interventional techniques like angioplasty ballooning, debulking, and stenting are not efficacious in the treatment of the crosslinked collagen in chronic clots due to the difficulty of removal of the clots without disrupting the vein walls to which they are attached.

Crosslinked collagen also forms as part of the build-up of venous scar tissue hindering Inferior Vena Cava (IVC) filter removal. In such therapies, an IVC filter is a device that is placed within the Inferior Vena Cava, the vein which carries blood from the low extremities back to the heart. When chronic collagenous clots in the veins of the legs and pelvis do become detached from the vein walls and travel to the lungs, they can cause a pulmonary embolism or blockage. IVC filters are implanted to help reduce the risk of pulmonary embolism by trapping large collagenous clots and preventing them from reaching the heart and lungs. However, where the IVC filter contacts the vein wall, collagenous clot-based scar tissue can build up, making IVC filter removal difficult and even impossible without disrupting the vein walls to which they are attached.

Crosslinked collagen also forms as part of the build-up of venous scar tissue hindering pacemaker and/or Implantable Cardioverter Defibrillator (ICD) removal. In such therapies, a pacemaker or ICD is implanted into a patient and wire leads are attached to the patient's heart. When needed, these wire leads deliver an electric shock from the pulse generator directly to the heart. When the wire leads need to be removed, however, the collagenous clot-based scar tissue that has built up around the leads makes the ICD removal difficult and even impossible without disrupting the vein walls to which they are attached. Inability to remove the leads percutaneously may result in the need for surgery.
CN 109223100 A, according to its abstract available in Espacenet, relates to treating cardiac valve and vascular calcification. The apparatus includes a shock wave transmitter for receiving a voltage/current pulse to generate a shock wave; a balloon sealingly connected to one end of the shock wave emitter such that an electrolyte liquid conducting the shock wave flows within the shock wave emitter and the balloon; a developing member located on the shock wave emitter and/or the balloon. The apparatus uses shock waves to soften the calcified area, and the treatment of the calcify area can be completed quickly and effectively.

Improvements in one or more aspects of the foregoing are desired.

### SUMMARY

The object of the present invention is solved by the subject-matter of the independent claim; further embodiments are incorporated in the dependent claims. Chronic clots and collagenous scar tissue are tough and rubbery obstructions found in the vasculature system. The physical dimensions and characteristics of these tissues resist traditional efforts to remove them and/or effectively open a lumen within their structure without disturbing the surrounding veins. Attempts to stent chronic clots often result in In Stent Thrombus (IST) or re-occlusion. Conventional techniques have not been extensively explored and can be expensive to do so. Surgical removal of chronic clots can be damaging to the surrounding tissues and still result in vessel scarring.

The systems according to the present disclosure generally relate to remodeling collagenous obstructions using energy and an expandable catheter. As opposed to using traditional techniques, these methods result in fewer complications, faster obstruction removal, and shorter recovery times. These and other advantages will be apparent from the disclosure of the aspects, examples, and configurations contained herein.

Method for treating a collagen obstruction within a subject's vasculature is presented for exemplification, the method comprising introducing a catheter into a subject's vasculature, the catheter comprising an expandable member and a conductive element adjacent to the expandable member, positioning the catheter adjacent to a collagen obstruction within the subject's vasculature, supplying energy to the conductive element, whereupon the energy reacts with water within the subject's vasculature and generates hydrogen ions that disrupt at least a portion of crosslinks within the collagen obstruction, and expanding the expandable member while the crosslinks are disrupted. In some examples, the method further includes inserting a guidewire through the collagen obstruction.

In some examples of the method, the collagen obstruction is a chronic clot or venous scar tissue.

In some examples of the method, the expandable member is a balloon catheter. In other examples, the expandable member is an expandable sheath.

In some examples of the method, supplying energy occurs prior to expanding the expandable member. In other examples of the method, supplying energy occurs simultaneous with expanding the expandable member. In some examples of the method, the supplying energy occurs for a predetermined interval of time. In some examples of the method, the energy is an electrical current or ultrasonic waves.

The present disclosure provides a system for treating a collagen obstruction within a subject's vasculature, the system comprising a catheter, the catheter comprising an expandable member and a conductive element adjacent to the expandable member, an electrical generator coupled to the conductive element, a control unit coupled to the electrical generator and/or the conductive element and catheter, wherein the control unit is configured to supply energy to the conductive element and causes expansion or retraction of the expandable member wherein the control unit comprises non-transitory computer-readable medium containing instructions that, when executed, cause one or more processors to supply 5-20 volts for a period of 1-5 minutes.

In some examples of the system, the expandable member is a balloon catheter. In some examples of the system, the expandable member is an expandable sheath.

In some examples, the system further comprises an inflation source coupled to the control system, wherein the control unit comprises non-transitory computer-readable medium containing instructions that, when executed, cause one or more processors to cause the electrical generator to supply energy to the conductive element prior to instructing the inflation source to expand the expandable member.

In some examples of the system the non-transitory computer-readable medium contains instructions that, when executed, cause one or more processors to cause the electrical generator to supply energy to the conductive element while simultaneously instructing the inflation source to expand the expandable member.

In some examples, the system further comprises an inflation source coupled to the control system, wherein the control unit comprises non-transitory computer-readable medium containing instructions that, when executed, cause one or more processors to cause the electrical generator to supply energy to the conductive element while simultaneously instructing the inflation source to expand the expandable member.

The preceding is a simplified summary of the disclosure to provide an understanding of some aspects of the disclosure. This summary is neither an extensive nor exhaustive overview of the disclosure and its various aspects, examples, and configurations. It is intended to present selected concepts of the disclosure in a simplified form as an introduction to the more detailed description presented below. As will be appreciated, other aspects, examples, and configurations of the disclosure are possible utilizing, alone or in combination, one or more of the features set forth above or described in detail below.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, examples, and configurations of the disclosure, as illustrated by the drawings referenced below.
FIGS. 1A-1C are schematic views of collagen: FIG. 1A depicts a collagen molecule; FIG. 1B depicts a collagen fibril; FIG. 1C depicts collagen fibers; and FIG. 1D is a photographic representation of a crosslinked collagen clot in the vasculature;
FIG. 2 is a flow diagram of a method for treating a collagen obstruction in accordance with a given example;
FIGS. 3A-3C are schematic views of a balloon system for use in accordance with examples of the present disclosure;
FIG. 4 is a schematic view of an expandable catheter in accordance with examples of the present disclosure; and
FIG. 5 is a schematic view of a system for treating a collagen obstruction in accordance with examples of the present disclosure.

### DETAILED DESCRIPTION

Systems according to the present disclosure generally relate to treating a collagen obstruction by remodeling collagenous clots or scar tissue using energy and an expandable catheter.

As used herein, "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and **Z₁-Zₒ,** the phrase is intended to refer to a single element selected from X, Y, and Z, a combination of elements selected from the same class (for example, X₁ and X₂) as well as a combination of elements selected from two or more classes (for example, Y₁ and Zₒ).

It is to be noted that the term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The terms "vasculature" and "vascular" as used herein refer to any part of the circulatory system of a subject, including peripheral and non-peripheral arteries and veins. Vascular material found within the vasculature can be comprised of both biological material (for example, nucleic acids, amino acids, carbohydrates, polysaccharides, lipids and the like) and non-biological material (for example, fat deposits, fibrous tissue, calcium deposits, remnants of dead cells, cellular debris and the like).

A "catheter" is a tube that can be inserted into a body cavity, duct, lumen, or vessel, such as the vasculature system. In most uses, a catheter is a relatively thin, flexible tube ("soft" catheter), though in some uses, it may be a larger, solid-less flexible-but possibly still flexible-catheter ("hard" catheter).

The term "balloon catheter" as used herein generally refers to the various types of angioplasty catheters which carry a balloon for performing angioplasty. Balloon catheters may also be of a wide variety of inner structure, such as different lumen design, of which there are at least three basic types: triple lumen, dual lumen and co-axial lumen. All varieties of internal structure and design variation are meant to be included by use of the term "balloon catheter" herein.

The term "electrically conductive" as used herein generally refers to a material having a measurable level of electrical conductivity, or the ability to support the movement of electrically charged particles.

It should be understood that every maximum numerical limitation given throughout this disclosure is deemed to include each and every lower numerical limitation as an alternative, as if such lower numerical limitations were expressly written herein. Every minimum numerical limitation given throughout this disclosure is deemed to include each and every higher numerical limitation as an alternative, as if such higher numerical limitations were expressly written herein. Every numerical range given throughout this disclosure is deemed to include each and every narrower numerical range that falls within such broader numerical range, as if such narrower numerical ranges were all expressly written herein.

Chronic venous disease is a condition in which clots form in the venous circulation system, especially in the lower extremities. A chronic clot, as used herein, refers to clots that are present for longer periods of time within the vasculature, greater than about 30 days. Chronic clots, like acute clots, can lead to a blockage or thrombosis of a blood vessel. In some cases, subjects having chronic clots, such as Deep Vein Thrombosis (DVT), may possess clots that have recannulated, allowing blood flow through the vein. Chronic clots are primarily formed of collagen fibers.

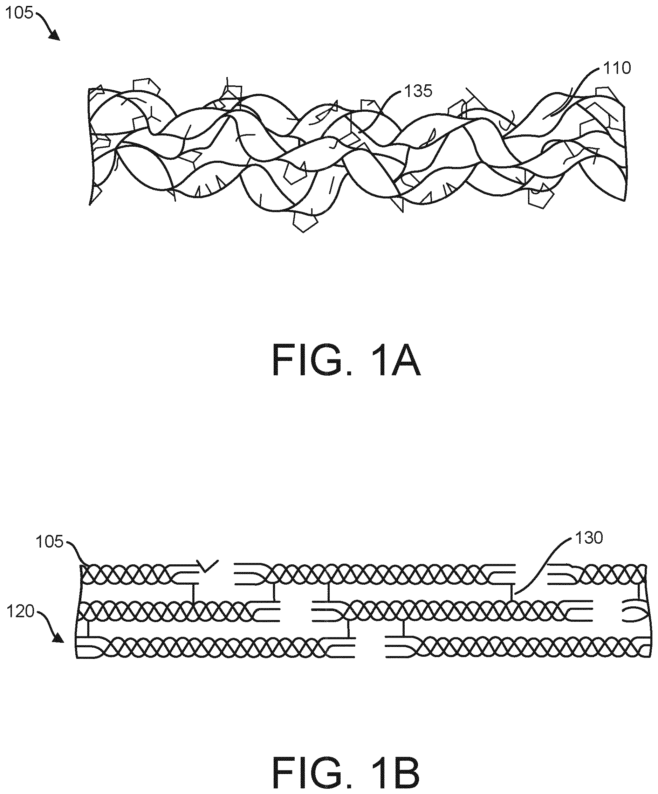

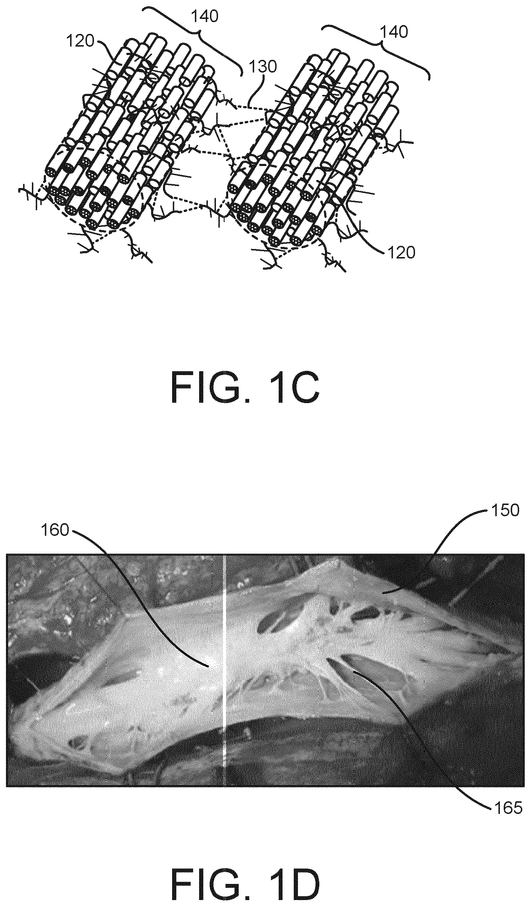

Collagen is the main structural protein in the extracellular matrix of various connective tissues and forms the basic structure of blood vessels. It is also a main component of wound healing. Referring now to FIGS. 1A-1D, particularly FIG. 1A, collagen molecules 105 are structurally formed by a triple helix of amino acids 110. Collagen molecules 105 are arranged such that the triple helices 110 are connected via a series of intramolecular crosslinks 135, with three helices 110 generating a single collagen molecule 105. As depicted in FIG. 1B, multiple collagen molecules 105 may be arranged together to form elongated collagen fibrils 120. In some configurations, the collagen molecules 105 are bound together via intermolecular crosslinks 130. As depicted in FIG. 1C, multiple collagen fibrils 120 may be arranged together to form elongated collagen fibers 140. In some instances, the collagen fibrils 120 are bound together via intermolecular crosslinks 130. These crosslinks 130 may take the form of ionic bonds, covalent bonds, disulfide bonds, and/or isopeptide bonds. Crosslinking increases the stiffness of collagen fibrils 120 and collagen fibers 140, and any resulting collagen clot. As depicted in FIG. 1D, crosslinked collagen may form a collagenous clot 160 within a peripheral vein of the vasculature 150. The collagenous clot 160 may be attached to the vasculature 150 via synechiae 165.

Though the physiochemical properties of collagen may be influenced by-among other factors-temperature, pH, salinity, and collagen concentration, in general, at a near-neutral or neutral pH (for example, pH 6-8), collagen molecules 105 self-assemble into collagen fibrils 120, which themselves self-assemble into collagen fibers 140, (FIG. 1A→FIG. 1B→FIG. 1C) via a series of intramolecular crosslinks 135 and intermolecular crosslinks 130. At an acidic pH, however, (for example, pH <6), these collagen crosslinks 130 are disrupted by the hydrogen ions, resulting in a disassembly (FIG. 1C→FIG. 1B→FIG. 1A) from collagen fibers 140 to collagen fibrils 120 to collagen molecules 105. Such an acidic pH may be created in a tissue via electrolysis of water, as detailed below.

The five most common types of collagen in the human body are known as Type I, Type II, Type III, Type IV, and Type V collagen. These groups are divided according to the structures they form, though all of the types of collagen contain at least one triple helix. Water is an integral component of collagen and contributes up to 60% (by weight) of the collagen. In forming collagen fibers, clots, and scar tissue, collagen may attach to other types of collagen molecules, as depicted in FIG. 1C, and other proteins, including but not limited to fibrin, glycoproteins, and/or proteoglycans. A collagen obstruction may attach to a vein wall via synechiae and/or fibronectin, laminin, fibulin, and/or integrin molecules.

This disclosure provides systems for a minimally invasive interventional treatment of a collagen obstruction, including a collagenous clot or scar tissue, and methods for exemplification are provided. The methods generally include the use of a conductive element within a catheter to target delivery of controlled amounts of hydrogen ions to the collagen obstruction. The generation of high local concentrations of hydrogen ions inside the collagen at the surface of the inserted electrode minimizes exposure of this acidic environment to the rest of the vasculature or local anatomy. Such a high local concentration of hydrogen ions may generate a pH range of 2-4. The rubbery, tough structure of a collagen obstruction softens after interaction with the hydrogen ions, eventually disrupting the collagen crosslinks and leaving more malleable, partially or fully uncrosslinked collagen fibers and/or fibrils. This softened tissue can then be physically remodeled using an expandable catheter (in the case of a clot) or the IVC filter or pacemaker/ICD lead can be removed entirely (in the case of scar tissue). Remodeling or removal results in the reopening of the lumen of the vein.

Once the conductive element discontinues supplying energy, hydrogen ions are no longer delivered to the site of therapy, and the natural buffering capacity within the vasculature will result in the collagen and surrounding tissues returning to a normal, balanced pH level. And upon the remaining collagen returning to a normal pH level, the remaining collagen will again self-assemble into crosslinks and become rubbery and resilient, but in a remodeled structure, rather than as an obstruction.

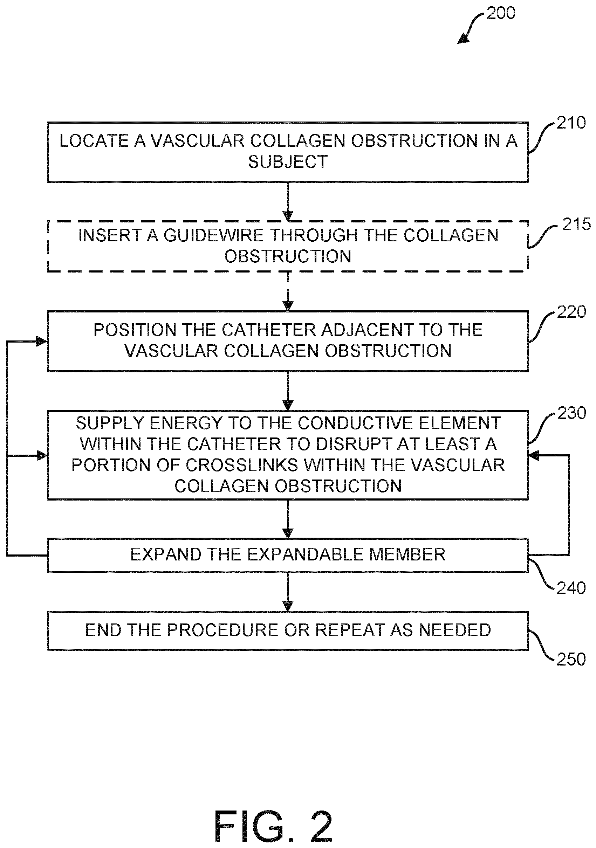

Referring to the flow chart in FIG. 2, specific materials and methods of the present examples include using energy and an expandable catheter to remodel collagen clots and/or collagen scar tissue. The present disclosure includes an example of method for treating a subject with a vascular collagen obstruction 200 using examples of the catheter described herein. Although it is not illustrated in FIG. 2, it may be desirable to use an atherectomy device, such as an ablation device, including a laser catheter to ablate at least a portion of the vascular collagen obstruction in the vessel of the subject prior to performing the method set forth in FIG. 2 and/or using the laser catheter to ablate at least a portion of the vascular collagen obstruction in the vessel prior to, during and/or subsequent to performing any of the steps set forth in FIG. 2.

The method 200 in FIG. 2 includes a step 210 of locating a vascular collagen obstruction in the vessel of a subject 210. The method 200 may optionally include a step 215 of inserting a guidewire through the collagen obstruction. The next step 220 includes positioning a catheter over the guidewire and adjacent to the vascular collagen obstruction. As will be discussed in more detail below, the catheter will include an expandable member and a conductive element. The method 200 next includes a step 230 of supplying energy to the conductive element coupled to the catheter to disrupt at least a portion of crosslinks within the vascular collagen obstruction 230. It may be desirable for the conductive element to produce 5-20 volts of electricity, for a period of up to 5 minutes. For example, the voltage may be 5, 6, 7, or 20 volts. For example, the cumulative period of time during which the electrical energy is applied may be about 1, 2, 3, 4 or 5 minutes. In other words, the electrical energy may be applied consecutively for the 1 to 5 minute time window, or the electrical energy may be applied in a series of smaller time windows that accumulate to the 1-5 minutes period. For example, the electrical energy may be applied for either 30 second, 1 minute, 90 seconds, 2 minutes, or 180 seconds sub-intervals for a cumulative period of 1-5 minutes. Supplying this amount of electricity for this period of time decreases the pH of the collagen to a pH level of less than 6, and in some situations to about pH 2 to pH 4. The amount and duration of electricity applied to the tissue depends on the depth of penetration into the tissue by the local concentrations of hydrogen ions, and in some instances the hydrogen ions may be located with 1 mm of the anode. Once the desired pH is obtained, it is desirable to maintain the reduced pH level for a certain period. Accordingly, the cumulative period(s) discussed above accomplish that goal.

The method 200 also includes the next step 240, which comprises expanding the expandable member of the catheter. Upon completing step 230 and/or 240, the catheter can optionally be repositioned within the vasculature. Upon completing step 240, step 230 may optionally be repeated. The method 200 may end at step 250 when the desired therapeutic outcome is obtained, or repeating any of 210 through 240, as may be necessary to treat a subject having a vascular collagen obstruction.

In some examples, the supplying energy in step 230 occurs prior to expanding the expandable member of the catheter in step 240. In other examples, the supplying energy in step 230 occurs simultaneous with expanding the expandable member of the catheter in step 240. In such examples, the amount of electricity supplied may be variable or may be constant, such that the pH of the collagen decreases to pH ≤6 and/or remains at pH ≤6.

In some examples, the disclosed method 200 may be used to deliver controlled amounts of hydrogen ions to a collagen obstruction located within the peripheral vasculature. For example, the methods may be applied to any vessel with a chronic clot, compressive disease, IST, or an implant with undesirable build-up of scar tissue. For example, the collagen obstruction may be located in a vein in the legs, feet, arms, or hands. In other specific examples, the methods may be used to deliver controlled amounts of hydrogen ions to the scar tissue on IVC filters to allow for easier/lower force removal of the IVC filters. In other examples, the disclosed methods may be used to deliver controlled amounts of hydrogen ions to the scar tissue on pacemaker/ICD leads to allow easier/lower force removal of the pacemaker/ICD leads.

In some examples, the method includes locating a vascular collagen obstruction in a subject and the percutaneous positioning the conductive element adjacent to a collagen obstruction using techniques similar to angioplasty. In some examples, the method includes using a venogram, and/or an ultrasound.

In some examples, the conductive element may have a first electrode, a second electrode, and an insulating layer between the first electrode and second electrode. The electrodes may be made of materials that can withstand high voltage levels and intense mechanical forces (for example, about 1000-2000 psi or 20-200 ATM in a few microseconds) that are generated during use. For example, the electrodes may be made of stainless steel, tungsten, nickel, iron, steel, and the like. The insulating layer may be made of any material with a high breakdown voltage, such as Kapton, ceramic, polyimide or Teflon.

In some examples, the conductive element may be an ultrasonically energized wire guide designed to provide a channel within a collagenous obstruction. In some examples, the current return electrode may be located on the skin or at some internal site. Alternatively, the negative (-) of the system could involve a non-insulated portion of the working guidewire or exposed portion of a conductive procedural sheath/guide.

In some examples, the conductive element may be supplied with electrical energy. For example, DC electric current may be used. In some aspects, the DC electric current may interact with water present in the collagen and produce hydrogen ions (also known as protons or represented herein as H+) via the anodic electrolysis at the surface of the conductive element, via the following reaction: H₂O → O₂ + 4H⁺ + 4e⁻. In other examples, AC electric current may be used. In some examples, the electrical energy may be controlled precisely by the amount of current applied from a constant current device. For example, the constant current device may be an IOMED Phoresor DC power source. In other examples, the dose of hydrogen ions may be controlled precisely by the amount of current applied from a variable current device.

In some examples, after the expandable member is expanded 240 the method may further include the optional step of reversing the polarity of the current, such that the current produces hydroxyl ions, which will neutralize the hydrogen ions previously produced to form water and restore collagen crosslinking. In some examples, this restoration of crosslinks may speed up or otherwise enhance the re-forming of the remodeled collagen.

In some examples, after the procedure ends 250 a stent or other physical device may be used to provide additional radial strength to the remodeled collagen. For example, particular compressive anatomical regions or disease profiles may benefit from such a device, including but not limited to, May-Thurner syndrome.

In some examples, the conductive element may be supplied with ultrasound energy. For example, the pressure waves of ultrasound may be used as the energy source in step 230. In other examples, the pressure waves of ultrasound may be used simultaneously with electrical energy in step 230, such that the ultrasound energy functions to drive the H+ ions deeper into the clot. This may increase the effectiveness of the method 200 because to the pressure waves disrupt the intraluminal as well as medial (within the tissue layer of the vascular wall) vascular obstructions (for example, calcium deposits), thereby creating a pathway for the energy to enter the collagen and disrupt the crosslinks. In some aspects, high frequency ultrasound could further relax the collagen through its mechanical disruption. In some aspects, the ultrasound vibration in combination with electrolysis of water at the surface may help distribute/diffuse hydrogen ions into the collagen more completely enhancing the effect.

In some examples, the energy may be applied for a predetermined period of time. In some examples, the energy may be applied in intermittent periods. For example, the energy may be pulsed on and off with the pulses lasting roughly 1 second, with a cumulative time of no more than a few minutes. For example, the energy may be applied for one or more periods lasting between 30 seconds and 10 minutes. In some aspects, the energy is applied for a cumulative time of at least about 1 minute, 2 minutes, 3 minutes, 4 minutes, 5 minutes, 6 minutes, 7 minutes, 8 minutes, 9 minutes, or 10 minutes.

The energy may be applied at a predetermined rate. For example, the energy may be electrical voltage or current, and may be applied at low levels. The energy may be applied at any value between 5-20 volts. Alternatively, in some examples, the energy may be applied at any voltage greater than the minimum voltage required for electrolysis of water is (~1.23 V), depending on the temperature and impurities in the water.

In some examples, the expandable member may be integrally formed with the conductive element such that the expandable member itself is electrified and/or capable of transmitting energy, and functions as the anode in producing hydrogen ions to the collagen obstruction. Using such a device may allow for increased wall apposition by allowing the expandable member to intrude into, or to sink into the collagen obstruction.

In other examples, the expandable member can physically press the conductive element or another electrically conductive material against another conductive material already in contact with the collagen obstruction. In such examples, the method may be used to induce an electrical potential between the conductive material already in contact with the collagen obstruction and another electrode (internal or external to the vessel) to drive the production of hydrogen ions into the collagen obstruction. For example, the conductive material already in contact with the collagen obstruction may be a stent. In other examples, the conductive material already in contact with the collagen obstruction may have undesirable scar tissue built up. For example, the conductive material may be an IVC filter, pacemaker/ICD leads, or the like.

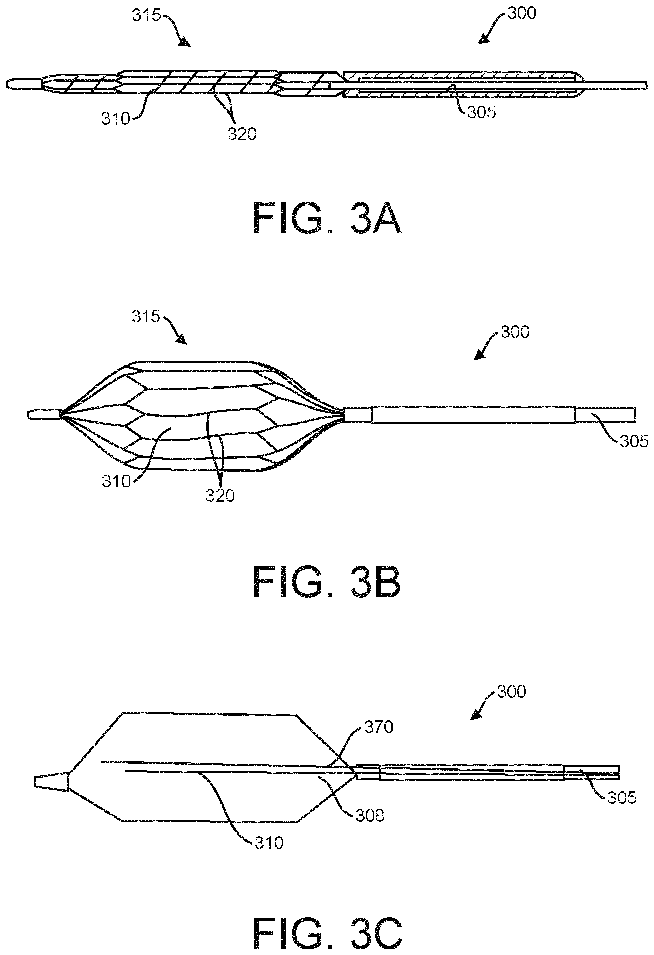

Several nonlimiting examples of the expandable member of the present disclosure are described further in FIGS. 3-4.

In some examples, the expandable member may be a balloon catheter. Referring now to FIGS. 3A-3C, a balloon catheter 300 of the present disclosure comprises a shaft 305 coupled to an inflatable balloon 310 and optionally an expansible shell or cage 315. The inflatable balloon 310 is located at the distal end of the catheter shaft 305 and configured to receive inflation medium from the inflation lumen in the shaft 305. In this way, the balloon 310 can alternate from a contracted or non-inflated configuration, as shown in FIG. 3A, to a fully inflated configuration, as shown in FIG. 3B. The expansible cage 315 may be circumferentially arranged around the outside of the inflatable balloon 310 so that the expansible cage 315 expands when balloon 310 inflates, as shown in FIG. 3B, and self-closes over the balloon, as shown in the contracted configuration of FIG. 3A. The expansible cage 315 is typically formed from a highly elastic metal, such as stainless steel, tantalum, platinum, cobalt chrome alloys, elgiloy or nitinol alloys, and may typically be formed by laser cutting of a hypo tube.

The balloon catheter 300 further includes a conductive element 370, which is or is coupled to the expansible cage 315. The elongate tubular body of the balloon catheter 300 defines an internal lumen 308 that may be configured and dimensioned to contain the conductive element 370 and, in some examples, a guide wire 310. At its proximal end and/or the proximal end the catheter 300, the conductive element 370 may be coupled to an electrical generator 530 and/or a control unit 575 used to supply energy to disrupt at least a portion of crosslinks within a collagen obstruction. In some examples, the balloon catheter 300 is uninflated during the positioning of the catheter in the vasculature and thereafter may be expanded to a partially or fully-inflated capacity, such that it expands to a functional diameter. In the functional diameter, it can achieve the desired therapeutic effect of physically remodeling the collagen while the collagen crosslinks are disrupted.

In some examples, the balloon catheter 300 may be expanded by inflating the balloon 310 with liquid medium. For example, the balloon 310 may be inflated with liquid medium to pressures greater than 0 atmospheres to about 20.0 atmospheres. Different degrees of force may be used or required to reshape collagen obstructions of different sizes, in different locations within the vasculature. The degree of force used or required to reshape each collagen obstruction, after the disruption of the crosslinks, will be appreciated by one of ordinary skill in the art based on the present disclosure. The use of dilation balloon catheters at low pressures can reduce the potential for damaging healthy vascular tissue during a procedure and can facilitate the treatment of a greater range and types of vascular obstructions.

In some examples, the balloon catheter 300 may be made of an electrically insulating material that may be rigid (for example, PET, etc.), semi-rigid (for example, PBAX, nylon, PEBA, polyethylene, etc.), or flexible (for example, polyurethane, silicone, etc.).

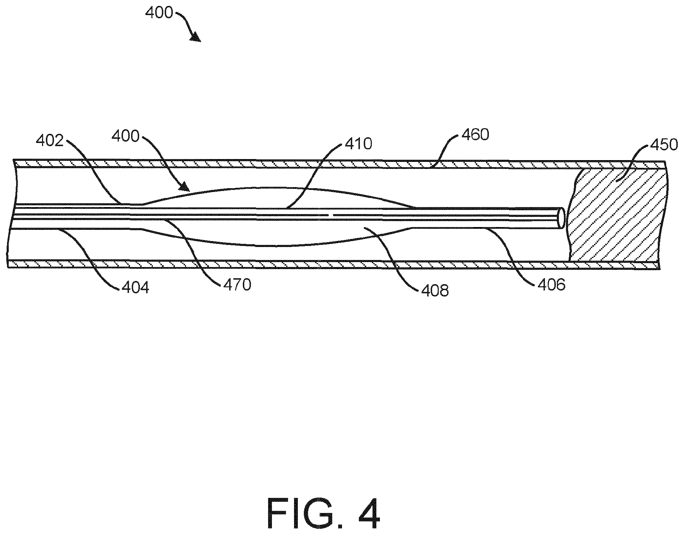

In some examples, the expandable member may be an expandable catheter. Referring now to FIG. 4 which illustrates one embodiment of a catheter 400 for use in the disclosed systems and the exemplary methods. In FIG. 4, a vascular collagen obstruction 450 is depicted within the vasculature 460. The catheter 400 includes an elongate tubular body 402 having a proximal region 404 and a distal region 406. The elongate tubular body 402 of the catheter 400 defines an internal lumen 408 that may be configured and dimensioned to contain a conductive element 470 and, in some examples, a guide wire 410. The conductive element 470 may be used to supply energy to disrupt at least a portion of crosslinks within a collagen obstruction 450. In some examples, the distal region 406 is configured to be expandable such that it may be radially collapsed during the positioning of the catheter in the vasculature and thereafter may be expanded such that it expands to its functional diameter. In the functional diameter, it can achieve the desired therapeutic effect of physically remodeling the collagen while the collagen crosslinks are disrupted.

In some examples, the expandable catheter may be expanded with a pressures greater than 0 atmospheres to about 20.0 atmospheres. Different degrees of force may be used or required to reshape collagen obstructions of different sizes, in different locations within the vasculature. The degree of force used or required to reshape each particular collagen obstruction, after the disruption of the crosslinks, will be appreciated by one of ordinary skill in the art based on the present disclosure.

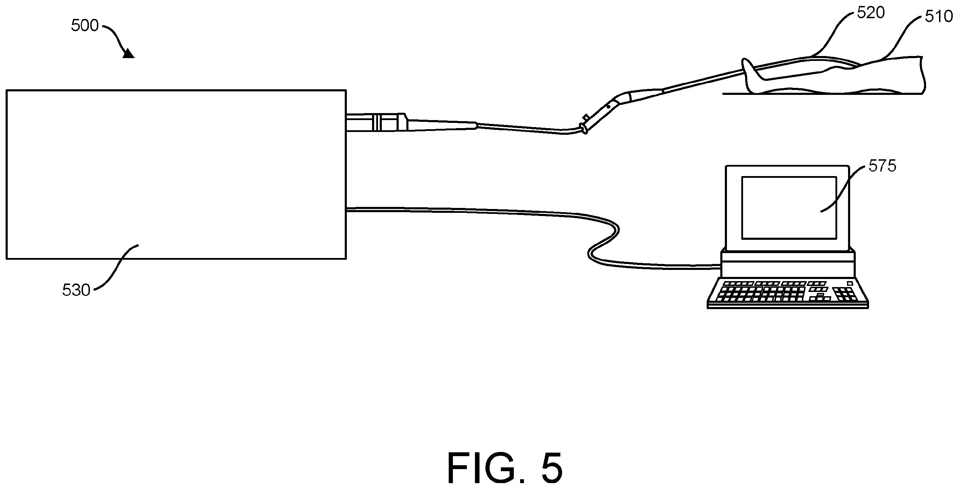

Referring now to FIG. 5, the methods described herein may be used as part of a catheter system 500 for treating a collagen obstruction in a subject 510. The catheter system 500 includes a catheter 520 coupled to an electrical generator 530, which is controlled by a control unit 575. The electrical generator 530 and/or control unit 575 includes controls to allow an operator to locate a collagen obstruction in a subject 510 and to adjust the location of the catheter 520 within the subject 510. The electrical generator 530 and/or control unit 575 includes controls to allow an operator to adjust the magnitude of the voltage, the current, the duration of the voltage, thereby controlling the duration and flow of the hydrogen ions. The electrical generator 530 and/or control unit 575 further includes controls to allow an operator to adjust the expansion and contraction of the expandable member.

The control unit 575 may be internal or external to the electrical generator 530, which may produce electrical pulses and/or charges. The control unit 575 may include one or more computing devices programmed to control the electrical generator 530. The generator 530 and/or control unit 575 may include a non-transitory computer-readable medium (for example, memory) that includes instructions that, when executed, cause one or more processors to control the electrical generator 530 and/or other components of the catheter system 500. For example, the non-transitory computer-readable medium shall include instructions to instruct the electrical generator 530 to apply the electricity having the selected voltage to the conductive element 370, 470 for the interval time windows and/or cumulative and/or consecutive periods discussed above. The control unit 575 may also be logically coupled to the inflation source (not shown) for inflating the expandable member. If so, the non-transitory computer-readable medium may also include instructions to instruct the electrical generator 530 to apply the electricity to the conductive element 370, 470 before, during and/or after the control unit 575 instructs the inflation source to inflate or expand the expandable member.

The control unit 575 may include one or more input devices to receive input from an operator. For example, input devices include keys, buttons, touch screens, dials, switches, mouse, and trackballs which may provide user control of the control unit 575. The control unit 575 may further include one or more output devices to provide feedback or information to an operator. For example, the output devices include a display, lights, audio devices which may provide user feedback or information.

The present disclosure, in various aspects, examples, and configurations, includes components, exemplary methods and processes, systems and/or apparatus substantially as depicted and described herein, including various aspects, examples, configurations, sub combinations, and subsets thereof. Those of skill in the art will understand how to make and use the various aspects, aspects, examples, and configurations, after understanding the present disclosure. The present disclosure, in various aspects, examples, and configurations, includes providing devices and processes in the absence of items not depicted and/or described herein or in various aspects, examples, and configurations hereof, including in the absence of such items as may have been used in previous devices or processes, for example, for improving performance, achieving ease and\or reducing cost of implementation.

Moreover, though the description of the disclosure has included description of one or more aspects, examples, or configurations and certain variations and modifications, other variations, combinations, and modifications are within the scope of the disclosure, for example, as may be within the skill and knowledge of those in the art, after understanding the present disclosure.

## Claims

1. A system (500) for treating a collagen obstruction within a subject's vasculature, the system comprising:
a catheter (520) comprising:
an expandable member and a conductive element (370, 470) adjacent to the expandable member, wherein the conductive element of the catheter is configured to be positioned adjacent to the collagen obstruction (450) within the subject's vasculature;
an electrical generator (530) coupled to the conductive element; and
a control unit (575) coupled to the electrical generator, wherein the control unit is configured to control supply of energy, from the electrical generator to the conductive element, to disrupt at least a portion of crosslinks within the collagen obstruction (450);
wherein the control unit comprises non-transitory computer-readable medium containing instructions that, when executed, cause one or more processors to supply 5-20 volts for a period of 1-5 minutes to the conductive element.

2. The system of claim 1, wherein the expandable member is a balloon catheter (300).

3. The system of claim 1, wherein the expandable member is an expandable sheath.

4. The system of claim 1, further comprising an inflation source coupled to the control system, wherein the control unit comprises non-transitory computer-readable medium containing instructions that, when executed, cause one or more processors to cause the electrical generator to supply energy to the conductive element prior to instructing the inflation source to expand the expandable member.

5. The system of claim 4, wherein the non-transitory computer-readable medium contains instructions that, when executed, cause one or more processors to cause the electrical generator to supply energy to the conductive element while simultaneously instructing the inflation source to expand the expandable member.

6. The system of claim 1, further comprising an inflation source coupled to the control system, wherein the control unit comprises non-transitory computer-readable medium containing instructions that, when executed, cause one or more processors to cause the electrical generator to supply energy to the conductive element while simultaneously instructing the inflation source to expand the expandable member.

7. The system of claim 1, wherein the system is configured for treating a collagen obstruction being a chronic clot or a venous scar tissue.

8. The system of claim 1, wherein the control unit is configured to supply energy to the conductive element prior to expanding the expandable member.

9. The system of claim 1, wherein the control unit is configured to supply energy to the conductive element simultaneously with expanding the expandable member.

10. The system of claim 1, wherein the control unit is configured to supply energy to the conductive element for a predetermined interval of time.

11. The system of claim 1, wherein the energy is an electrical current or ultrasonic waves.

12. The system of claim 1, wherein the conductive element is configured to be supplied with ultrasound energy simultaneously with electrical energy.

## Patentansprüche

1. System (500) zur Behandlung einer Kollagenobstruktion innerhalb des Gefäßsystems eines Subjekts, wobei das System Folgendes umfasst:
einen Katheter (520), umfassend:
ein expandierbares Element und ein leitfähiges Element (370, 470) angrenzend an das expandierbare Element, wobei das leitfähige Element des Katheters konfiguriert ist, um angrenzend an die Kollagenobstruktion (450) innerhalb des Gefäßsystems des Patienten positioniert zu werden;
einen mit dem leitfähigen Element gekoppelten elektrischen Generator (530); und
eine Steuereinheit (575), die mit dem elektrischen Generator gekoppelt ist, wobei die Steuereinheit konfiguriert ist, um die Energiezufuhr vom elektrischen Generator zum leitfähigen Element zu steuern, um mindestens einen Teil der Vernetzungen innerhalb der Kollagenobstruktion (450) zu unterbrechen;
wobei die Steuereinheit ein nichtflüchtiges, computerlesbares Medium umfasst, das Anweisungen enthält, die bei Ausführung einen oder mehrere Prozessoren veranlassen, dem leitfähigen Element für einen Zeitraum von 1 bis 5 Minuten 5 bis 20 Volt zuzuführen.

2. System nach Anspruch 1, wobei das expandierbare Element ein Ballonkatheter (300) ist.

3. System nach Anspruch 1, wobei das expandierbare Element eine expandierbare Hülle ist.

4. System nach Anspruch 1, weiter umfassend eine mit dem Steuersystem gekoppelte Inflationsquelle, wobei die Steuereinheit ein nichtflüchtiges, computerlesbares Medium umfasst, das Anweisungen enthält, die bei Ausführung einen oder mehrere Prozessoren veranlassen, den elektrischen Generator zu veranlassen, dem leitfähigen Element Energie zuzuführen, bevor die Inflationsquelle angewiesen wird, das expandierbare Element zu expandieren.

5. System nach Anspruch 4, wobei das nichtflüchtige, computerlesbare Medium Anweisungen enthält, die bei Ausführung einen oder mehrere Prozessoren veranlassen, den elektrischen Generator zu veranlassen, dem leitfähigen Element Energie zuzuführen, während gleichzeitig die Inflationsquelle angewiesen wird, das expandierbare Element zu expandieren.

6. System nach Anspruch 1, weiter umfassend eine mit dem Steuersystem gekoppelte Inflationsquelle, wobei die Steuereinheit ein nichtflüchtiges, computerlesbares Medium umfasst, das Anweisungen enthält, die bei Ausführung einen oder mehrere Prozessoren veranlassen, den elektrischen Generator zu veranlassen, dem leitfähigen Element Energie zuzuführen, während die Inflationsquelle angewiesen wird, das expandierbare Element zu expandieren.

7. System nach Anspruch 1, wobei das System zur Behandlung einer Kollagenobstruktion, die ein chronisches Gerinnsel oder venöses Narbengewebe ist, konfiguriert ist.

8. System nach Anspruch 1, wobei die Steuereinheit konfiguriert ist, um dem leitfähigen Element vor dem Expandieren des expandierbaren Elements Energie zuzuführen.

9. System nach Anspruch 1, wobei die Steuereinheit konfiguriert ist, um gleichzeitig mit dem Expandieren des expandierbaren Elements dem leitfähigen Element Energie zuzuführen.

10. System nach Anspruch 1, wobei die Steuereinheit konfiguriert ist, um dem leitfähigen Element für einen vorbestimmten Zeitraum Energie zuführen.

11. System nach Anspruch 1, wobei die Energie ein elektrischer Strom oder Ultraschallwellen ist.

12. System nach Anspruch 1, wobei das leitfähige Element zur Zufuhr von Ultraschallenergie gleichzeitig mit elektrischer Energie konfiguriert ist.

## Revendications

1. Système (500) pour le traitement d'une obstruction de collagène dans le système vasculaire d'un sujet, le système comprenant :
un cathéter (520) comprenant :
un organe extensible et un élément conducteur (370, 470) adjacent à l'organe extensible, dans lequel l'élément conducteur du cathéter est configuré pour être positionné de manière adjacente à l'obstruction de collagène (450) dans le système vasculaire du sujet ;
un générateur électrique (530) couplé à l'élément conducteur ; et
une unité de commande (575) couplée au générateur électrique, dans lequel l'unité de commande est configurée pour commander l'alimentation en énergie, du générateur électrique à l'élément conducteur, afin de perturber au moins une partie des liaisons croisées au sein de l'obstruction de collagène (450) ;
dans lequel l'unité de commande comprend un support non transitoire lisible par ordinateur contenant des instructions qui, lorsqu'elles sont exécutées, amènent un ou plusieurs processeurs à fournir 5-20 volts pendant une période de 1-5 minutes à l'élément conducteur.

2. Système selon la revendication 1, dans lequel l'organe extensible est un cathéter à ballonnet (300).

3. Système selon la revendication 1, dans lequel l'organe extensible est une gaine extensible.

4. Système selon la revendication 1, comprenant en outre une source de gonflage couplée au système de commande, dans lequel l'unité de commande comprend un support non transitoire lisible par ordinateur contenant des instructions qui, lorsqu'elles sont exécutées, amènent un ou plusieurs processeurs à faire en sorte que le générateur électrique fournisse de l'énergie à l'élément conducteur avant d'ordonner à la source de gonflage de dilater l'organe extensible.

5. Système selon la revendication 4, dans lequel le support non transitoire lisible par ordinateur contient des instructions qui, lorsqu'elles sont exécutées, amènent un ou plusieurs processeurs à faire en sorte que le générateur électrique fournisse de l'énergie à l'élément conducteur tout en ordonnant simultanément à la source de gonflage d'étendre l'organe extensible.

6. Système selon la revendication 1, comprenant en outre une source de gonflage couplée au système de commande, dans lequel l'unité de commande comprend un support non transitoire lisible par ordinateur contenant des instructions qui, lorsqu'elles sont exécutées, amènent un ou plusieurs processeurs à faire en sorte que le générateur électrique fournisse de l'énergie à l'élément conducteur tout en ordonnant simultanément à la source de gonflage d'étendre l'organe extensible.

7. Système selon la revendication 1, dans lequel le système est configuré pour traiter une obstruction de collagène étant un caillot chronique ou un tissu cicatriciel veineux.

8. Système selon la revendication 1, dans lequel l'unité de commande est configurée pour fournir de l'énergie à l'élément conducteur avant d'étendre l'organe extensible.

9. Système selon la revendication 1, dans lequel l'unité de commande est configurée pour fournir de l'énergie à l'élément conducteur simultanément à l'expansion de l'organe extensible.

10. Système selon la revendication 1, dans lequel l'unité de commande est configurée pour fournir de l'énergie à l'élément conducteur pendant un intervalle de temps prédéterminé.

11. Système selon la revendication 1, dans lequel l'énergie est un courant électrique ou des ondes ultrasonores.

12. Système selon la revendication 1, dans lequel l'élément conducteur est configuré pour être alimenté en énergie ultrasonore simultanément avec de l'énergie électrique.
